# EUROPÄISCHE PATENTSCHRIFT

(11) **EP 3 749 375 B1**
(45) Veröffentlichungstag und Bekanntmachung des Hinweises auf die Patenterteilung: **26.10.2022**
(21) Anmeldenummer: 19700887.3
(22) Anmeldetag: 14.01.2019
(51) Int. Cl.: A61L 2/07, A61L 2/28, F28F 27/00, F28B 9/10, F22B 37/38, F25B 43/04

(54) **VERFAHREN UND VORRICHTUNG ZUM ERKENNEN EINES NICHTKONDENSIERBAREN ANTEILS EINES ZUMINDEST TEILWEISE GASFÖRMIG VORLIEGENDEN MEDIUMS**
METHOD AND DEVICE FOR DETECTING A NON-CONDENSABLE PORTION OF AN AT LEAST PARTIALLY GASEOUS MEDIUM
PROCÉDÉ ET DISPOSITIF DE DÉTECTION DU FRACTION NON CONDENSABLE D'UNE SUBSTANCE AU MOINS PARTIELLEMENT GAZEUSE

(30) Priorität: 06.02.2018 DE 102018102631
(43) Veröffentlichungstag der Anmeldung: 16.12.2020
(73) Patentinhaber: Endress + Hauser Flowtec AG, 4153 Reinach (CH)
(72) Erfinder: HÖCKER, Rainer, 79761 Waldshut (DE)
(74) Vertreter: Waselikowski, Stefan
(86) Internationale Anmeldenummer: PCT/EP2019/050808
(87) Internationale Veröffentlichungsnummer: WO 2019/154593

(56) Entgegenhaltungen:
- EP-A1- 1 715 302
- EP-A1- 1 882 479
- EP-A1- 2 851 679
- EP-A2- 3 088 862
- EP-B1- 0 762 902
- DE-A1- 19 718 347
- US-A- 3 234 748
- US-A- 3 967 494
- US-A- 4 739 647
- US-A- 5 752 411

## Beschreibung

Die Erfindung betrifft ein Verfahren und eine Vorrichtung zum Erkennen eines unerwünschten nichtkondensierbaren Anteils eines gasförmig vorliegenden Mediums.

Gasförmige kondensierbare, also dampfförmig vorliegende Medien werden in der Technik für verschiedene Zwecke eingesetzt. So kann beispielsweise Dampf dazu genutzt werden, über einen Wärmetauscher Energie an ein anderes Medium zu übertragen. Dampf, insbesondere Wasserdampf kann auch zur Sterilisation von beispielsweise medizinischen Geräten verwendet werden, da eine Kondensation des Wasserdampfs große Energiemengen freisetzt, welche unerwünschte Keime zuverlässig abtötet.

In solchen Fällen sind nichtkondensierbare gasförmige Anteile im Dampf wie beispielsweise Luft unerwünscht, da es die Funktion von Wärmetauschern beeinträchtigt und die Sterilisation von Keimen unter Umständen nur unvollständig wäre.

Aus dem Stand der Technik sind Vorrichtungen bekannt, mittels welcher ein nichtkondensierbarer Anteil in einem gasförmig vorliegenden Medium erkannt und abgeschieden werden kann.

So offenbart das Dokument US 3 234 748 einen Kältekreislauf, bei dem der Gehalt an nichtkondensierbaren Gasen im Kältemitteldampf bestimmt wird. Dabei werden Temperatur und Druck ermittelt, ein Verhältnis von Druck zu Temperatur gebildet und dieses Verhältnis mit einem Sollwert-Verhältnis verglichen. Die Offenlegungsschrift DE 197 18 347 A1 zeigt einen Dampferzeuger und ein Verfahren zur Reinigung von Wasserdampf, insbesondere zur Abscheidung von nichtkondensierbaren Gasen, wobei Temperatur und Druck gemessen werden, um den Sattdampfzustand zu ermitteln und mit theoretischen Sattdampfbedingungen zu vergleichen.

Aus der EP 2 851 679 A1 ist eine Vorrichtung gemäß dem Oberbegriff des Anspruchs 9 und ein entsprechendes Verfahren zur Ermittlung des Gehaltes an nichtkondensierbaren Gasen in einem Dampfstrom bekannt, bei dem neben der Temperatur und dem Druck auch ein Volumenstrom zur Bestimmung des Gasvolumens ermittelt wird.

Aufgabe der Erfindung ist es daher, ein Verfahren und eine Vorrichtung vorzuschlagen, mittels welcher ein nichtkondensierbarer Anteil in einem gasförmig vorliegenden Medium noch besser und effektiver erkannt werden kann.

Die Aufgabe wird gelöst durch ein Verfahren gemäß dem unabhängigen Anspruch 1 sowie durch eine Vorrichtung gemäß dem unabhängigen Anspruch 9.

Bei einem erfindungsgemäßen Verfahren zum Erkennen eines nichtkondensierbaren Anteils eines Mediums, welches Medium zumindest einen kondensierbaren Anteil aufweist und gasförmig vorliegt,
misst in einem ersten Verfahrensschritt ein Temperaturmessgerät eine Temperatur des Mediums, wobei ein Druckmessgerät einen Druck des Mediums misst,
wobei in einem zweiten Verfahrensschritt mittels einer elektronischen Mess-/Betriebsschaltung ein Verhältnis des Drucks zur Temperatur gebildet wird, und dieses Verhältnis mit einem Sollverhältnis verglichen wird,
wobei die elektronische Mess-/Betriebsschaltung im Falle einer Mindestabweichung des Verhältnisses vom Sollverhältnis in einem dritten Verfahrensschritt eine Meldung ausgibt.

Das gasförmige Medium kann feine kondensierte Tröpfchen tragen bzw. mitführen, also nebelartig sein und kann sich dabei in einem thermodynamischen Gleichgewicht befinden, so dass es gesättigt ist. Zusätzlich zur Nebelartigkeit des Mediums kann es zu einer Ausbildung eines Flüssigkeitsfilms oder eines Bodensatzes an einer Wandung des Behältnisses bzw. der Rohrleitung kommen. Nichtkondensierbar bedeutet in diesem Zusammenhang, dass der nichtkondensierbare Anteil bei Anwendung des erfindungsgemäßen Verfahren nicht kondensiert. So kann beispielsweise bei einem Luft-Wasserdampf-Gemisch der Luftanteil bei entsprechender Abkühlung auch kondensiert werden, jedoch wird bei einem Wärmetauscher, welcher mit Wasserdampf arbeitet bei sachgemäßem Gebrauch eine Siedetemperatur eines wesentlichen Luftbestandteils unter keinen Umständen erreicht. Insofern ist Luft im Sinne der Erfindung nicht kondensierbar. Entsprechendes gilt für weitere erfindungsgemäße Verfahren.

Erfindungsgemäß strömt das Medium durch eine Rohrleitung. Beispielsweise kann die Rohrleitung Teil eines Wärmekraftwerks oder eines Kühlschranks sein.

Beispielsweise ist die Rohrleitung Teil eines Sterilisationsgeräts.

Erfindungsgemäß misst ein Durchflussmessgerät einen Durchfluss des Mediums durch die Rohrleitung, wobei die elektronische Mess-/Betriebsschaltung auf Basis des gemessenen Durchflusses sowie der einer Mindestabweichung des Verhältnisses einen Durchfluss des kondensierbaren Anteils ermittelt, wobei die elektronische Mess-/Betriebsschaltung dem Durchflussmessgerät zugeordnet ist.

In technischen Geräten, welche auf einem Wärmetauschprinzip basieren, kann somit die Wärmetauschleistung korrigiert bzw. ermittelt werden. Bei Sterilisationsgeräten kann somit eine Sterilisationsleistung angegeben werden.

In einer Ausgestaltung wird mittels eines Verifikationsmessgeräts das Vorliegen eines gasförmigen Mediums verifiziert, wobei das Verifikationsmessgerät eine Strömungsgeschwindigkeit des Mediums in der Rohrleitung und/oder mindestens eine der folgenden Medieneigenschaften misst:
Schallgeschwindigkeit, Schallabsorption, Absorption elektromagnetischer Strahlung, elektrische Leitfähigkeit, Dichte, Viskosität, Wärmeleitfähigkeit, Amplitude einer Oberflächenwelle einer Innenwandung des Behältnisses bzw. der Rohrleitung, Frequenz einer Oberflächenwelle der Innenwandung des Behältnisses bzw. der Rohrleitung, Phase einer Oberflächenwelle der Innenwandung des Behältnisses bzw. der Rohrleitung,
wobei bei einer Mindestabweichung einer der gemessenen Medieneigenschaften von einem erwarteten Sollwert bzw. Sollwertbereich auf das Vorliegen eines flüssigen Mediums geschlossen wird.

Oberflächenwellen einer Innenwandung des Behältnisses oder einer Innenwandung der Rohrleitung werden dabei durch Eigenschaften des im Behältnis bzw. in der Rohrleitung befindlichen Mediums beeinflusst und können somit zur Verifikation des Vorliegens eines gasförmigen Mediums verwendet werden.

Der im zweiten Verfahrensschritt durchgeführte Vergleich des Verhältnisses des Drucks zur Temperatur mit einem Sollverhältnis kann auch bei Vorliegen eines flüssigen Mediums eine Mindestabweichung erkennen. In diesem Fall kann mittels einer Verifikation eine Fehlfunktion oder eine Fehlinterpretation verhindert werden.

In einer Ausgestaltung weist das Verifikationsmessgerät eine Sendevorrichtung und eine Empfangsvorrichtung zum Aussenden bzw. Empfangen von akustischen Signalen oder elektromagnetischen Signalen auf.

In einer Ausgestaltung ist das Durchflussmessgerät ein Ultraschall-Durchflussmessgerät, ein thermisches Massedurchflussmessgerät, ein Vortex-Durchflussmessgerät oder ein Coriolis-Durchflussmessgerät.

In einer Ausgestaltung ist das Ultraschall-Durchflussmessgerät bzw. das Coriolis-Durchflussmessgerät das Verifikationsmessgerät.

In einer Ausgestaltung weist der kondensierbare Anteil des Mediums bei einem Arbeitsdruck einen Siedepunkt auf, welcher um mindestens 20 K und insbesondere mindestens 40 K und bevorzugt mindestens 60 K höher ist, als ein Siedepunkt des nichtkondensierbaren Anteils.

In einer Ausgestaltung weist der kondensierbare Anteil zumindest eines der folgenden Substanzen bzw. zumindest eine Substanz aus mindestens einer der folgenden Substanzgruppen auf:
Wasser, Kohlenwasserstoff, Alkohol, Ammoniak, Kältemittel gemäß DIN-8960 Ausgabe 1998-11.

In einer Ausgestaltung führt die Rohrleitung das Medium zu einem Wärmetauscher, welcher Wärmetauscher eine Kondensation des kondensierbaren Anteils zum Wärmetauschen zwischen dem Medium und einem weiteren Medium nutzt.

Eine erfindungsgemäße Vorrichtung zum Erkennen eines nichtkondensierbaren Anteils eines Mediums, welches Medium zumindest einen kondensierbaren Anteil aufweist und in der Rohrleitung gasförmig vorliegt, wobei die Vorrichtung dazu eingerichtet ist, ein Verfahren gemäß einem der vorigen Ansprüche umzusetzen, umfasst:
ein Temperaturmessgerät, welches dazu eingerichtet ist, die Temperatur des Mediums zu messen;
ein Druckmessgerät, welches dazu eingerichtet ist, den Druck des Mediums zu messen;
ein Verifikationsmessgerät, welches dazu eingerichtet ist, das Vorliegen eines gasförmigen Zustands des Mediums zu verifizieren,
eine elektronische Mess-/Betriebsschaltung, welche dazu eingerichtet ist, das Temperaturmessgerät, das Druckmessgerät sowie das Verifikationsmessgerät zu betreiben, wobei die elektronische Mess-/Betriebsschaltung dazu eingerichtet ist, bei einer Mindestabweichung einer der gemessenen Medieneigenschaften von einem erwarteten Sollwert bzw. Sollwertbereich auf das Vorliegen eines flüssigen Mediums zu schließen.

In einer Ausgestaltung umfasst die Vorrichtung ein Durchflussmessgerät, welches stromabwärts oder stromaufwärts zu einer Rohrleitung angeordnet ist, wobei das Durchflussmessgerät ein Messrohr aufweist, welches Messrohr dazu eingerichtet ist, an die Rohrleitung angeschlossen zu werden.

In einer Ausgestaltung ist das Verifikationsmessgerät dazu eingerichtet, eine Strömungsgeschwindigkeit des Mediums in der Rohrleitung und/oder mindestens eines der folgenden Medieneigenschaften zu messen:
Schallgeschwindigkeit, Schallabsorption, Absorption elektromagnetischer Strahlung, elektrische Leitfähigkeit, Dichte, Viskosität, Wärmeleitfähigkeit.

In einer Ausgestaltung ist das Durchflussmessgerät ein Ultraschall-Durchflussmessgerät, ein thermisches Massedurchflussmessgerät, ein Vortex-Durchflussmessgerät oder ein Coriolis-Durchflussmessgerät.

In einer Ausgestaltung ist das Durchflussmessgerät ein Ultraschall-Durchflussmessgerät oder ein Coriolis-Durchflussmessgerät und umfasst das Verifikationsmessgerät.

Im Folgenden wird die Erfindung anhand von Ausführungsbeispielen beschrieben:

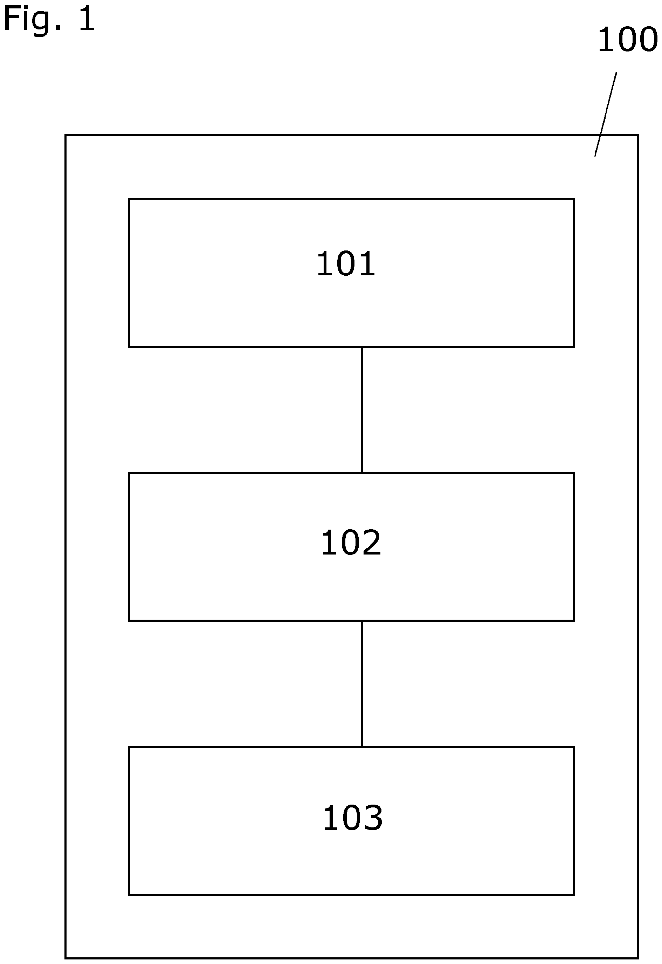

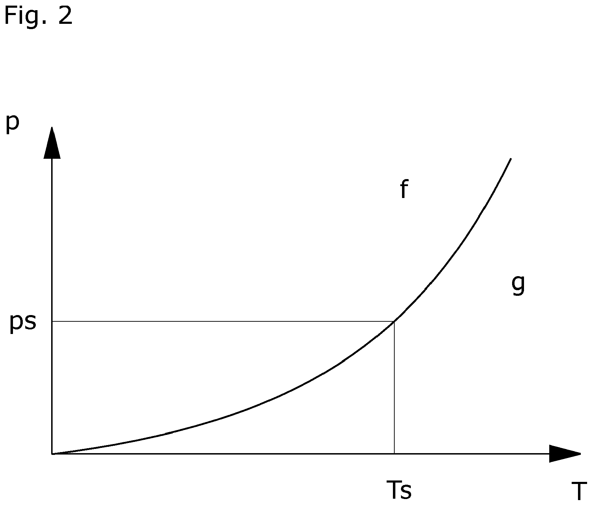

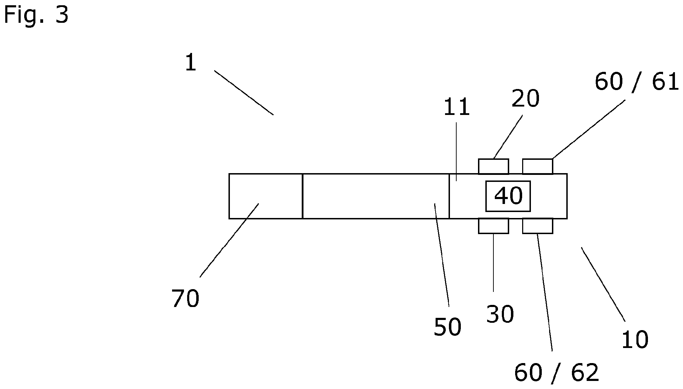

Fig. 1 beschreibt ein erfindungsgemäßes Verfahren; und Fig. 2 skizziert den erfinderischen Gedanken anhand eines Ausschnitts aus einem Phasendiagramm eines kondensierbaren Anteils eines beispielhaften Mediums; und Fig. 3 skizziert eine beispielhafte erfindungsgemäße Vorrichtung.

Fig. 1 beschreibt ein erfindungsgemäßes Verfahren 100, wobei zum Erkennen eines nichtkondensierbaren Anteils eines Mediums in einem ersten Verfahrensschritt 101 ein Temperaturmessgerät 20 eine Temperatur des Mediums misst, und wobei ein Druckmessgerät 30 einen Druck des Mediums misst. In einem zweiten Verfahrensschritt 102 bildet eine elektronische Mess-/Betriebsschaltung 40 aus dem gemessenen Druck und der gemessenen Temperatur ein Verhältnis und vergleicht dieses Verhältnis mit einem Sollverhältnis. Bei einem Medium, bei welchem ein kondensierbarer Anteil gesättigt ist, kann über eine Sattdampfdruckkurve über einen gemessenen Druck eine Solltemperatur bzw. über eine gemessene Temperatur ein Solldruck berechnet werden und somit ein Sollverhältnis ermittelt werden. Durch Rohrleitungen strömt ein gesättigtes zumindest teilweise gasförmig vorliegendes Medium dabei sehr häufig dann, wenn das Medium durch Sieden eines kondensierten Vorrats des Mediums durch den dabei entstehenden Dampfdruck in die Rohrleitung eingeleitet wird. Da bei technischen Vorrichtungen aus vielen Gründen keine vollständige Abgeschlossenheit gegenüber einer Außenwelt zu gewährleisten ist, kann das Medium auch einen störenden nichtkondensierbaren Anteil aufweisen.

Für den Fall, dass ein aus der gemessenen Temperatur und dem gemessenen Druck ermitteltes Verhältnis eine Mindestabweichung zum Sollverhältnis festgestellt wird, gibt die elektronische Mess-/Betriebsschaltung 40 in einem dritten Verfahrensschritt eine Meldung aus, dass das Medium eine Verunreinigung durch einen nichtkondensierbaren Anteil aufweist.

An die Rohrleitung kann ein Durchflussmessgerät 10 angeschlossen sein, welches den Durchfluss des Mediums durch die Rohrleitung 50 misst, wobei die elektronische Mess-/Betriebsschaltung auf Basis des gemessenen Durchflusses sowie der Abweichung einen Durchfluss des kondensierbaren Anteils ermittelt. Auf diese Weise kann beispielsweise die Wärmeübertragungsleistung eines an die Rohrleitung angeschlossenen Wärmeübertragers besser ermittelt werden.

Um sicherzustellen, dass beispielsweise aufgrund von technischen Fehlern keine Flüssigkeit durch die Rohrleitung strömt, kann ein Verifikationsmessgerät 60 eingesetzt werden, welches den Aggregatzustand des Mediums bestimmt. Beispielsweise kann das Verifikationsmessgerät eine Durchflussmessung vornehmen oder Medieneigenschaften wie beispielsweise Schallgeschwindigkeit, Schallabsorption, Absorption elektromagnetischer Strahlung, elektrische Leitfähigkeit, Dichte, Viskosität Wärmeleitfähigkeit, Amplitude einer Oberflächenwelle, Frequenz einer Oberflächenwelle oder Phase einer Oberflächenwelle bestimmen und anhand eines bestimmten Werts der entsprechenden Medieneigenschaft das Vorliegen eines zumindest teilweise gasförmigen Zustands verifizieren. Das Verifikationsmessgerät kann beispielsweise eine Sendevorrichtung 61 und eine Empfangsvorrichtung 62 zum Aussenden bzw. Empfangen von akustischen Signalen oder elektromagnetischen Signalen aufweisen. Beispielsweise kann ein Ultraschall-Durchflussmessgerät oder ein Coriolis-Durchflussmessgerät die Funktion des Verifikationsmessgeräts übernehmen. Fig. 2 skizziert ein beispielhaftes Phasendiagramm eines Mediums wie beispielsweise Wasser oder einem Kohlenwasserstoff oder einem Alkohol oder einem Gemisch aus mindestens einem der vorgenannten Stoffe, wobei ein Druck p und eine Temperatur T des Mediums in einem gesättigten Zustand durch eine Sättigungsdampfdruckkurve gegeben sind, welche Sättigungsdampfdruckkurve in einem Druck-Temperatur-Diagramm eine Grenze zwischen einem flüssigen Aggregatzustand und einem gasförmigen Aggregatzustand ist.

Würde bei einer Messung des Drucks des Mediums gleichzeitig eine geringere Temperatur als eine über die Sättigungsdampfdruckkurve erwartete Solltemperatur Ts gemessen werden, kann dies als Vorliegen eines nichtkondensierbaren Anteils im Medium interpretiert werden. Entsprechend kann falls bei einer Messung der Temperatur gleichzeitig ein höherer Druck als ein erwarteter Solldruck ps vorliegt auch auf das Vorliegen eines nichtkondensierbaren Anteils im Medium interpretiert werden. Bei entsprechender Interpretation kann ein Volumenanteil des nichtkondensierbaren Anteils am Gesamtvolumen bestimmt werden.

Fig. 3 skizziert eine beispielhafte Vorrichtung umfassend ein Durchflussmessgerät 10, ein Temperaturmessgerät 20, ein Druckmessgerät 30 und ein Verifikationsmessgerät 60, wobei die Vorrichtung mit einer Rohrleitung 50 verbunden ist, welche dazu eingerichtet ist, ein durch die Rohrleitung strömendes Medium zu einem Wärmetauscher zu leiten. Das Durchflussmessgerät weist ein Messrohr 11 auf, welches das Medium zur Rohrleitung leitet. Das Temperaturmessgerät 20 und das Druckmessgerät 30 sind dabei am bzw. im Messrohr angeordnet. Eine elektronische Mess-/Betriebsschaltung 40, welche dazu eingerichtet ist, das Temperaturmessgerät, das Druckmessgerät sowie das Verifikationsmessgerät zu betreiben, ist Bestandteil des Durchflussmessgeräts. Alternativ kann die elektronische Mess-/Betriebsschaltung aber auch eine vom Durchflussmessgerät losgelöste Schaltung sein. Beispielsweise wie hier gezeigt, kann das Verifikationsmessgerät 60 eine Sendevorrichtung 61 und eine Empfangsvorrichtung 62 für das Senden und Empfangen von akustischen Signalen oder elektromagnetischen Signalen aufweisen, welche am oder im Messrohr angeordnet sind. Im Falle, dass das Durchflussmessgerät ein Ultraschall-Durchflussmessgerät ist, kann das Ultraschall-Durchflussmessgerät die Funktion des Verifikationsmessgeräts übernehmen. Alternativ oder zusätzlich kann die Sendevorrichtung und die Empfangsvorrichtung auch in einem Bereich der Rohrleitung angeordnet sein.

### Bezugszeichenliste

- 1: Vorrichtung
- 10: Durchflussmessgerät
- 11: Messrohr
- 20: Temperaturmessgerät
- 30: Druckmessgerät
- 40: elektronische Mess-/Betriebsschaltung
- 50: Rohrleitung
- 60: Verifikationsmessgerät
- 61: Sendevorrichtung
- 62: Empfangsvorrichtung
- 70: Wärmetauscher
- ps: Solldruck
- Ts: Solltemperatur
- 100: Verfahren
- 101: erster Verfahrensschritt
- 102: zweiter Verfahrensschritt
- 103: dritter Verfahrensschritt

## Patentansprüche

1. Verfahren (100) zum Erkennen eines nichtkondensierbaren Anteils eines Mediums, welches Medium zumindest einen kondensierbaren Anteil aufweist und gasförmig vorliegt,
wobei in einem ersten Verfahrensschritt (101) ein Temperaturmessgerät (20) eine Temperatur des Mediums misst, und wobei ein Druckmessgerät (30) einen Druck des Mediums misst,
wobei in einem zweiten Verfahrensschritt (102) mittels einer elektronischen Mess-/Betriebsschaltung (40) ein Verhältnis des Drucks zur Temperatur gebildet wird, und dieses Verhältnis mit einem Sollverhältnis aus einem Solldruck (ps) und einer Solltemperatur (Ts) verglichen wird,
wobei die elektronische Mess-/Betriebsschaltung (40) im Falle einer Mindestabweichung
des Verhältnisses vom Sollverhältnis in einem dritten Verfahrensschritt (103) eine Meldung ausgibt,
wobei das Medium durch eine Rohrleitung (50) strömt,
wobei ein Durchflussmessgerät (10) einen Durchfluss des Mediums durch die Rohrleitung (50) misst, wobei die elektronische Mess-/Betriebsschaltung (40) auf Basis des
gemessenen Durchflusses sowie der Abweichung einen Durchfluss des kondensierbaren Anteils ermittelt, wobei die elektronische Mess-/Betriebsschaltung (40) dem Durchflussmessgerät (10) zugeordnet ist.

2. Verfahren nach Anspruch 1,
wobei mittels eines Verifikationsmessgeräts (60) das Vorliegen eines gasförmigen Mediums verifiziert wird, wobei das Verifikationsmessgerät (60) eine Strömungsgeschwindigkeit des Mediums in der Rohrleitung (50)
und/oder mindestens
eine der folgenden Medieneigenschaften misst:
Schallgeschwindigkeit, Schallabsorption, Absorption elektromagnetischer Strahlung, elektrische Leitfähigkeit, Dichte, Viskosität, Wärmeleitfähigkeit, Amplitude einer Oberflächenwelle, Frequenz einer Oberflächenwelle, Phase einer Oberflächenwelle wobei bei einer Mindestabweichung einer der gemessenen Medieneigenschaften von einem erwarteten Sollwert oder
Sollwertbereich auf das Vorliegen eines flüssigen Mediums geschlossen wird.

3. Verfahren nach Anspruch 2,
wobei das Verifikationsmessgerät (60) eine Sendevorrichtung (61) und eine Empfangsvorrichtung (62) zum Aussenden bzw. Empfangen von akustischen Signalen oder elektromagnetischen Signalen aufweist.

4. Verfahren nach einem der Ansprüche 1 bis 3,
wobei das Durchflussmessgerät (60) ein Ultraschall-Durchflussmessgerät, ein thermisches Massedurchflussmessgerät, ein Vortex-Durchflussmessgerät oder ein Coriolis-Durchflussmessgerät ist.

5. Verfahren nach Anspruch 4,
wobei das Ultraschall-Durchflussmessgerät bzw. das Coriolis-Durchflussmessgerät das Verifikationsmessgerät (60) ist.

6. Verfahren nach einem der vorigen Ansprüche,
wobei der kondensierbare Anteil des Mediums bei einem Arbeitsdruck einen Siedepunkt aufweist, welcher um mindestens 20 K und insbesondere mindestens 40 K und bevorzugt mindestens 60 K höher ist, als ein Siedepunkt des nichtkondensierbaren Anteils.

7. Verfahren nach einem der vorigen Ansprüche,
wobei der kondensierbare Anteil zumindest eine der folgenden Substanzen oder zumindest eine Substanz aus einer der folgenden Substanzgruppen aufweist:
Wasser, Kohlenwasserstoff, Alkohol, Ammoniak, Kältemittel gemäß DIN-8960 Ausgabe 1998-11.

8. Verfahren nach einem der Ansprüche 1 bis 7,
wobei die Rohrleitung (50) das Medium zu einem Wärmetauscher (70) führt, welcher Wärmetauscher (70) eine Kondensation des kondensierbaren Anteils zum Wärmetauschen zwischen dem Medium und einem weiteren Medium nutzt.

9. Vorrichtung (1) zum Erkennen eines nichtkondensierbaren Anteils eines Mediums, welches Medium zumindest einen kondensierbaren Anteil aufweist und zumindest teilweise gasförmig vorliegt, wobei die Vorrichtung (1) umfasst:
ein Temperaturmessgerät (20), welche dazu eingerichtet ist, die Temperatur des Mediums zu messen;
ein Druckmessgerät (30), welche dazu eingerichtet ist, den Druck des Mediums zu messen;
ein Verifikationsmessgerät (60), welches dazu eingerichtet ist, das Vorliegen eines gasförmigen Zustands des Mediums zu verifizieren,
eine elektronische Mess-/Betriebsschaltung (40), welche dazu eingerichtet ist, das Temperaturmessgerät (20), das Druckmessgerät (30) sowie das Verifikationsmessgerät (60) zu betreiben, wobei die elektronische Mess-/Betriebsschaltung (40) dazu eingerichtet ist, bei einer Mindestabweichung einer der gemessenen Medieneigenschaften von einem erwarteten Sollwert oder
Sollwertbereich auf das Vorliegen eines flüssigen Mediums zu schließen, **dadurch gekennzeichnet, dass**
die Vorrichtung (10) dazu eingerichtet ist, ein Verfahren (100) gemäß einem der vorigen Ansprüche umzusetzen.

10. Vorrichtung nach Anspruch 9,
wobei die Vorrichtung ein Durchflussmessgerät (10) umfasst, welches stromabwärts oder stromaufwärts zu einer Rohrleitung (50) angeordnet ist,
wobei das Durchflussmessgerät (10) ein Messrohr (11) aufweist, welches Messrohr (11) dazu eingerichtet ist, an die Rohrleitung (50) angeschlossen zu werden.

11. Vorrichtung nach Anspruch 9 oder 10,
wobei das Verifikationsmessgerät (60) dazu eingerichtet ist, eine Strömungsgeschwindigkeit des Mediums in der Rohrleitung (50) und/oder mindestens
eines der folgenden Medieneigenschaften zu messen:
Schallgeschwindigkeit, Schallabsorption, Absorption elektromagnetischer Strahlung, elektrische Leitfähigkeit, Dichte, Viskosität, Wärmeleitfähigkeit, Amplitude einer Oberflächenwelle einer Innenwandung der Rohrleitung, Frequenz einer Oberflächenwelle der Innenwandung der Rohrleitung, Phase einer Oberflächenwelle der Innenwandung der Rohrleitung.

12. Vorrichtung nach Anspruch 10 oder 11,
wobei das Durchflussmessgerät (10) ein Ultraschall-Durchflussmessgerät, ein thermisches Massedurchflussmessgerät, ein Vortex-Durchflussmessgerät oder ein Coriolis-Durchflussmessgerät ist.

13. Vorrichtung nach Anspruch 12,
wobei das Durchflussmessgerät (10) ein Ultraschall-Durchflussmessgerät oder ein Coriolis-Durchflussmessgerät ist und das Verifikationsmessgerät (60) umfasst

## Claims

1. Procedure (100) for detecting a non-condensable part of a medium, said medium having at least one condensable part and being present in gaseous form, wherein, in a first procedural step (101), a temperature measuring device (20) measures a temperature of the medium and a pressure measuring device (30) measures a pressure of the medium,
wherein, in a second procedural step (102), a ratio of the pressure to the temperature is formed using an electronic measuring/operating circuit (40), and said ratio is compared to a target ratio consisting of a target pressure (ps) and a target temperature (Ts),
wherein, in the event of a minimum deviation of the ratio from the target ratio, the electronic measuring/operating circuit (40) outputs a message in a third procedural step (103),
wherein the medium flows through a pipe (50),
wherein a flowmeter (10) measures a flow of the medium through the pipe (50),
wherein the electronic measuring/operating circuit (40) determines a flow of the condensable part on the basis of the measured flow and the deviation, wherein the electronic measuring/operating circuit (40) is assigned to the flowmeter (10).

2. Procedure as claimed in Claim 1,
wherein the presence of a gaseous medium is verified by a verification measuring device (60), wherein the verification measuring device (60) measures a flow velocity of the medium through the pipe (50) and/or measures at least one of the following medium properties:
sound velocity, sound absorption, absorption of electromagnetic radiation,
electrical conductivity, density, viscosity, thermal conductivity, amplitude of a surface wave, frequency of a surface wave, phase of a surface wave,
wherein the presence of a liquid medium is deduced in the event of a minimum deviation of one of the measured medium properties from an expected target value or a target value range.

3. Procedure as claimed in Claim 2,
wherein the verification measuring device (60) comprises a transmission unit (61) and a reception unit (62) to transmit and receive acoustic signals or electromagnetic signals.

4. Procedure as claimed in one of the Claims 1 to 3,
wherein the flowmeter (60) is an ultrasonic flowmeter, a thermal mass flowmeter, a vortex flowmeter or a Coriolis flowmeter.

5. Procedure as claimed in Claim 4,
wherein the ultrasonic flowmeter or the Coriolis flowmeter is the verification measuring device (60).

6. Procedure as claimed in one of the previous claims,
wherein, at a working pressure, the condensable part of the medium has a boiling point that is at least 20 K and particularly at least 40 K and preferably at least 60 K higher than a boiling point of the non-condensable part.

7. Procedure as claimed in one of the previous claims,
wherein the condensable part comprises at least one of the following substances or at least one substance from one of the following substance groups: water, hydrocarbon, alcohol, ammoniac, refrigerant according to DIN-8960 edition 1998-11.

8. Procedure as claimed in one of the Claims 1 to 7,
wherein the pipe (50) conducts the medium to a heat exchanger (70), wherein said heat exchanger (70) uses a condensation of the condensable part to exchange heat between the medium and another medium.

9. Device (1) designed to detect a non-condensable part of a medium, said medium having at least one condensable part and being present at least partially in gaseous form, said device (1) comprising:
a temperature measuring device (20), which is designed to measure the temperature of the medium;
a pressure measuring device (30), which is designed to measure the pressure of the medium;
a verification measuring device (60), which is designed to verify the presence of a gaseous state of the medium,
an electronic measuring/operating circuit (40), which is designed to operate the temperature measuring device (20), the pressure measuring device (30) and the verification measuring device (60), wherein the electronic measuring/operating circuit (40) is designed to conclude the presence of a liquid medium in the event of a minimum deviation of one of the measured medium properties from an expected target value or an expected target value range,
**characterized in that**
the device (10) is designed to implement a procedure (100) as claimed in one of the previous claims.

10. Device as claimed in Claim 9,
wherein the device comprises a flowmeter (10), which is arranged upstream or downstream from a pipe (50), wherein the flowmeter (10) comprises a measuring tube (11), wherein the measuring tube (11) is designed to be connected to the pipe (50).

11. Device as claimed in Claim 9 or 10,
wherein the verification measuring device (60) is designed to measure a flow velocity of the medium in the pipe (50) and/or at least one of the following medium properties:
sound velocity, sound absorption, absorption of electromagnetic radiation,
electrical conductivity, density, viscosity, thermal conductivity, amplitude of a surface wave of an internal wall of the pipe, frequency of a surface wave of the internal wall of the pipe, phase of a surface wave of the internal wall of the pipe.

12. Device as claimed in Claim 10 or 11,
wherein the flowmeter (10) is an ultrasonic flowmeter, a thermal mass flowmeter, a vortex flowmeter or a Coriolis flowmeter.

13. Device as claimed in Claim 12,
wherein the flowmeter (10) is an ultrasonic flowmeter or a Coriolis flowmeter and
comprises the verification measuring device (60).

## Revendications

1. Procédé (100) destiné à la détection d'une partie non condensable d'un produit, lequel produit présente au moins une partie condensable et se présente sous forme gazeuse,
procédé pour lequel, dans une première étape de procédé (101), un appareil de mesure de température (20) mesure une température du produit et un appareil de mesure de pression (30) mesure une pression du produit,
procédé pour lequel, dans une deuxième étape de procédé (102), un rapport de la pression à la température est formé au moyen d'un circuit électronique de mesure/d'exploitation (40), et ce rapport est comparé à un rapport de consigne constitué d'une pression de consigne (ps) et d'une température de consigne (Ts), le circuit électronique de mesure/d'exploitation (40) émettant un message dans le cas d'un écart minimum entre le rapport et le rapport de consigne dans une troisième étape de procédé (103),
le produit s'écoulant à travers une conduite (50),
un débitmètre (10) mesurant un débit du produit à travers la conduite (50), le circuit électronique de mesure/d'exploitation (40) déterminant un débit de la partie condensable sur la base du débit mesuré ainsi que de l'écart, le circuit électronique de mesure/d'exploitation (40) étant associé au débitmètre (10).

2. Procédé selon la revendication 1,
pour lequel la présence d'un produit gazeux est vérifiée au moyen d'un appareil de mesure de vérification (60), l'appareil de mesure de vérification (60) mesurant une vitesse d'écoulement du produit dans la conduite (50) et/ou au moins l'une des propriétés de produit suivantes :
vitesse du son, absorption du son, absorption du rayonnement électromagnétique, conductivité électrique, densité, viscosité, conductivité thermique, amplitude d'une onde de surface, fréquence d'une onde de surface,
phase d'une onde de surface,
la présence d'un produit liquide étant déduite en cas d'écart minimal de l'une des propriétés de produit mesurées par rapport à une valeur de consigne ou une gamme de valeurs de consigne attendue.

3. Procédé selon la revendication 2,
pour lequel l'appareil de mesure de vérification (60) comprend un dispositif d'émission (61) et un dispositif de réception (62) pour émettre et recevoir des signaux acoustiques ou des signaux électromagnétiques.

4. Procédé selon la revendication 1 à 3,
pour lequel le débitmètre (60) est un débitmètre à ultrasons, un débitmètre massique thermique, un débitmètre vortex ou un débitmètre Coriolis.

5. Procédé selon la revendication 4,
pour lequel le débitmètre à ultrasons ou le débitmètre Coriolis est l'appareil de mesure de vérification (60).

6. Procédé selon l'une des revendications précédentes,
pour lequel la partie condensable du produit présente, à une pression de travail, un point d'ébullition qui est supérieur d'au moins 20 K et notamment d'au moins 40 K et de préférence d'au moins 60 K à un point d'ébullition de la partie non condensable.

7. Procédé selon l'une des revendications précédentes,
pour lequel la partie condensable comprenant au moins une des substances suivantes ou au moins une substance appartenant à l'un des groupes de substances suivants : eau, hydrocarbure, alcool, ammoniac, agent réfrigérant selon DIN-8960 édition 1998-11.

8. Procédé selon l'une des revendications 1 à 7,
pour lequel la conduite (50) amène le produit à un échangeur de chaleur (70), lequel échangeur de chaleur (70) utilise une condensation de la partie condensable pour échanger de la chaleur entre le produit et un autre produit.

9. Dispositif (1) destiné à la détection d'une partie non condensable d'un produit, lequel produit présente au moins une partie condensable et se présente au moins partiellement sous forme gazeuse, le dispositif (1) comprenant :
un appareil de mesure de la température (20), lequel est conçu pour mesurer la température du produit ;
un appareil de mesure de la pression (30), lequel est conçu pour mesurer la pression du produit ;
un appareil de mesure de vérification (60), lequel est conçu pour vérifier la présence d'un état gazeux du produit,
un circuit électronique de mesure/d'exploitation (40), lequel est conçu pour exploiter l'appareil de mesure de température (20), l'appareil de mesure de pression (30) ainsi que l'appareil de mesure de vérification (60), le circuit électronique de mesure/d'exploitation (40) étant conçu pour conclure à la présence d'un produit liquide en cas d'écart minimal de l'une des propriétés de produit mesurées par rapport à une valeur de consigne ou une gamme de valeurs de consigne attendue,
**caractérisé en ce que**
le dispositif (10) est conçu pour mettre en œuvre un procédé (100) selon l'une des revendications précédentes.

10. Dispositif selon la revendication 9,
pour lequel le dispositif comprend un débitmètre (10), lequel débitmètre est disposé en aval ou en amont d'une conduite (50), le débitmètre (10) comprenant un tube de mesure (11), lequel tube de mesure (11) est conçu pour être raccordé à la conduite (50).

11. Dispositif selon la revendication 9 ou 10,
pour lequel l'appareil de mesure de vérification (60) est conçu pour mesurer une vitesse d'écoulement du produit dans la conduite (50) et/ou au moins l'une des propriétés de produit suivantes :
vitesse du son, absorption du son, absorption du rayonnement électromagnétique, conductivité électrique, densité, viscosité, conductivité thermique, amplitude d'une onde de surface d'une paroi interne de la conduite, fréquence d'une onde de surface de la paroi interne de la conduite, phase d'une onde de surface de la paroi interne de la conduite.

12. Dispositif selon la revendication 10 ou 11,
pour lequel le débitmètre (10) est un débitmètre à ultrasons, un débitmètre massique thermique, un débitmètre vortex ou un débitmètre Coriolis.

13. Dispositif selon la revendication 12,
pour lequel le débitmètre (10) est un débitmètre à ultrasons ou un débitmètre Coriolis et comprend l'appareil de mesure de vérification (60).
